# EUROPEAN PATENT SPECIFICATION

(11) **EP 3 452 436 B1**
(45) Date of publication and mention of the grant of the patent: **13.10.2021**
(21) Application number: 17787615.8
(22) Date of filing: 05.05.2017
(51) Int. Cl.: C07B 59/00, A61K 51/08

(54) **IN-KIT PREPARATION OF GALLIUM-68 LABELLED RADIOPHARMACEUTICALS**
IN-KIT-HERSTELLUNG VON GALLIUM-68-MARKIERTEN RADIOPHARMAKA
PRÉPARATION EN KIT DE PRODUITS RADIO-PHARMACEUTIQUES MARQUÉS AU GALLIUM-68

(30) Priority: 06.05.2016 IT UA20163207
(43) Date of publication of application: 13.03.2019
(73) Proprietor: Gamma Servizi S.r.l., 27010 Borgarello (Pavia) (IT)
(72) Inventor: ASTI, Mattia, 42025 Cavriago (Reggio Emilia) (IT); IORI, Michele, 42033 Carpineti (Reggio Emilia) (IT); CAPPONI, Pier Cesare, 42123 Reggio Emilia (IT); RUBAGOTTI, Sara, 42122 Reggio Emilia (IT)
(74) Representative: Biggi, Cristina
(86) International application number: PCT/IB2017/052621
(87) International publication number: WO 2017/191604

(56) References cited:
- LINJING MU ET AL: "Identification, characterization and suppression of side-products formed during the synthesis of high dose 68Ga-DOTA-TATE", APPLIED RADIATION AND ISOTOPES., vol. 76, 1 June 2013 (2013-06-01), pages 63-69, XP055305221, GB ISSN: 0969-8043, DOI: 10.1016/j.apradiso.2012.07.022
- MATTIA ASTI ET AL.: "Development of a simple kit-bases method for preparation of pharmaceutical grade 68Ga-DOTATOC", NUCLEAR MEDICINE COMMUNICATIONS, vol. 36, no. 5, 2015, pages 502-510, XP000081765, cited in the application
- Ornchuma Naksuriya ET AL: "Comparison and combination effects on antioxidant power of curcumin with gallic acid, ascorbic acid, and xanthone", DD&T Drug Discoveries & Therapeutics, vol. 9, no. 2, 1 January 2015 (2015-01-01) , pages 136-141, XP55688397, Japan ISSN: 1881-7831, DOI: 10.5582/ddt.2015.01013
- TWOROWSKA IZABELA ET AL: "Radiosynthesis of clinical doses of68Ga-DOTATATE (GalioMedix(TM)) and validation of organic-matrix-based68Ge/68Ga generators", NUCLEAR MEDICINE AND BIOLOGY, vol. 43, no. 1, 1 January 2016 (2016-01-01), pages 19-26, XP029352766, ISSN: 0969-8051, DOI: 10.1016/J.NUCMEDBIO.2015.08.004

## Description

### TECHNICAL FIELD

The present invention relates to a process and a kit for the preparation of complexes of the ⁶⁸Ga radioisotope. In particular, the invention relates to a method for preparing molecules labelled with the ⁶⁸Ga radioactive isotope, useful as radiotracers and/or radiopharmaceuticals for the diagnosis and/or the treatment of specific diseases.

### BACKGROUND OF THE INVENTION

Radiotracers are molecules used in the diagnostic imaging field, for example in positron emission tomography (PET) and in single photon emission computed tomography (SPECT) for diagnosing the presence of diseases, in particular tumours.

A radiotracer consists of a molecule - which shows affinity for a specific molecular receptor which, for example, is over-expressed by tumour cells - covalently bonded to a chelating agent able to complex a radioactive isotope, for example ⁶⁸Ga, ⁹⁰Y and ¹⁷⁷Lu.

The use of radiotracers in diagnostic techniques has been very successful especially for the diagnosis (and also the treatment) of neuroendocrine tumours that express somatostatin receptors through radiolabelling with ⁶⁸Ga, ⁹⁰Y or ¹⁷⁷Lu of peptide analogues of somatostatin showing a high affinity for somatostatin receptors. This high affinity allows the peptide analogues of somatostatin to accumulate in the tumour cells that over-express such receptors. The most common peptide analogues of somatostatin used for these diagnostic applications are TOC, TATE and NOC, which are normally bonded, by means of an amide bond, through their carboxylic residue, to chelating molecules, the most common of which are 1,4,7,10-tetraacetic acid-1,4,7,10-tetraazacyclododecane (DOTA) and diethylenetriaminepentaacetic acid (DTPA).

Other applications that use radiolabelling technology regard, for example, the diagnosis of tumours that over-express the receptor of the human gastrin releasing peptide (GRP). Tumours that over-express the GRP receptor (rGRP) are prostate, breast, lung and pancreatic cancers. The radiotracer used for the diagnosis of these diseases normally consists of a GRP analogue, bombesin (BBN) (a peptide of 14 amino acids that shows affinity for the rGRP receptor), covalently bonded to DOTA, the chelating agent of the ⁶⁸Ga or ¹⁷⁷Lu radionucleotides.

The PET technique has numerous advantages with respect to the SPECT technique in terms of spatial resolution and sensitivity. For this reason, positron-emitting radionucleotides, for example ⁶⁸Ga, are preferred over γ-emitting radionucleotides for labelling peptides or other molecules. Among positron emitters, ⁶⁸Ga is normally the most commonly used since it has a positron energy and a half life suitable for applications in diagnostic nuclear medicine. Furthermore, ⁶⁸Ga can be obtained by ⁶⁸Ge/⁶⁸Ga generators in a chemical form and a purity suitable for labelling molecules, in particular peptides.

The possibility to use ⁶⁸Ge/⁶⁸Ga generators represents a great advantage for nuclear medicine since it avoids the need to possess a cyclotron.

The disadvantage of using ⁶⁸Ge/⁶⁸Ga generators regards the radiopharmaceutical quality of ⁶⁸Ga eluates that often is not sufficient for their direct use on patients or, however, for their use as radioactive precursors. The purity problems depend on the high acidity of the eluate and the presence in the ⁶⁸Ga eluate of metal impurities and/or of ⁶⁸Ge, which can compete with ⁶⁸Ga during the complexation reaction.

The contaminants present in the ⁶⁸Ga eluate do not allow the direct use of the eluate in the subsequent complexation reaction, without a prior purification.

Kits for radiolabelling peptides are known in the field, that allow to avoid purifying the eluate at the outlet of the generator and, therefore, allow the complexation reaction to be performed directly.

For example, Linjing Mu et al., Applied Radiation and Isotopes, vol. 76, 2013-06-01, p. 63-69, disclose a process wherein the ⁶⁸Ga complexation occurs in the presence of a neutralising agent (i.e. sodium-acetate buffer) and ascorbic acid. A salt of Ga³⁺ (i.e. gallium nitrate, Ga(NO₃)₃) is added only at the end of the complexation reaction for analytical purposes to determine the side-products formed during the synthesis of ⁶⁸Ga-DOTA-TATE. Gallium nitrate reacts with the excess of the chelator-functionalised molecule (which is in large excess with respect to the amount of ⁶⁸Ga) thus forming impurities that can be analysed by means of mass spectroscopy. In this way, the authors determine the level of impurities present at the end of the complexation reaction and find that the use of ascorbic acid positively affects the reduction of the amounts of impurities. Tworowska et al., Nuclear Medicine and Biology 43 (2016), p. 19-26, disclose a process for making ⁶⁸Ga complexes by eluting ⁶⁸Ga from the generator directly into a vial for use in a further reaction with a ligand. The vial comprises sodium acetate (as neutralizing agent) and ascorbic acid for the stabilization of the product. The complexation reaction described by the authors is possible because a purification method of the eluate is put into place before the complexation reaction, which allows the removal of a large part of the metal impurities and the yield of a ⁶⁸Ga solution with higher purity.

As far as ascorbic acid is concerned, Naksuriya et al., Drug Discoveries & Therapeutics 2015, 9(2), p. 136-141 investigated the antioxidant activity of curcumin alone and/or in combination with other natural antioxidants: gallic acid, ascorbic acid and xanthone. The results showed by these authors indicated that, as far as the antioxidant activity is concerned, curcumin revealed synergistic antioxidant effect when combined with gallic acid whereas an antagonist effect occurred when combined with ascorbic acid or xanthone.

Another example of kits for radiolabelling peptides is provided by Asti et al. Nuclear Medicine Communication, 2015, vol. 36, n. 5, p. 502-510, which describe a kit for radiolabelling DOTATOC wherein the ⁶⁸Ga eluate obtained from the generator is collected directly, without a prior purification, in a container containing ascorbic acid and sodium formate as a buffer to keep the pH at about 3.3. After heating the mixture to 100°C, sodium ascorbate is added to bring the pH to 5.5. The results obtained with this first attempt to produce a radiolabelling kit without intermediate purifications were positive since the purity of the solution after the labelling reaction of peptides with ⁶⁸Ga was slightly lower, but still comparable, to that obtained with commercial synthesisers.

Despite the good results obtained with this first kit attempt, in the field there is still a need to develop a process for radiolabelling molecules of diagnostic and therapeutic interest, and a related radiolabelling kit, which can offer better performance levels with respect to known processes and kits, in terms of lower formation of subproducts during the molecule labelling reaction and in terms of selectivity and chelating yield of ⁶⁸Ga.

### SUMMARY OF THE INVENTION

The scope of the present invention is only defined by the appended claims. The description is used for illustration purposes.

The present invention relates to a process for the preparation of complexes of the ⁶⁸Ga radioisotope in which the complexation reaction between a chelator-functionalised molecule and ⁶⁸Ga occurs in the presence of a neutralising agent, ascorbic acid and/or salts thereof, a salt of Ga³⁺ and/or curcumin or a derivative thereof.

The neutralising agent may be an alkaline or alkaline-earth salt of formic acid or ascorbic acid.

The process according to the invention also comprises an acid elution step of ⁶⁸Ga from a commercial generator directly into the solution containing a chelator-functionalised molecule, a neutralising agent, ascorbic acid and/or salts thereof, a salt of Ga³⁺and/or curcumin or a derivative thereof. In particular, the eluate obtained from the commercial generator of ⁶⁸Ga is added to a solution as above without performing an intermediate purification step.

The process according to the invention preferably comprises a step of adding a salt of ascorbic acid at the end of the complexation reaction.

In one embodiment, in the process for the preparation of complexes of the ⁶⁸Ga radioisotope, the complexation reaction between a chelator-functionalised molecule and ⁶⁸Ga occurs in the presence of an alkaline or alkaline-earth salt of ascorbic acid (preferably sodium ascorbate) as the neutralising agent. In this case, curcumin and/or the gallium salt may be present in the reaction mixture or they may be absent. Furthermore, it is possible for ascorbic acid not to be added to the reaction mixture since it is formed, in part, following the dissociation of the ascorbic acid salt in solution.

The ⁶⁸Ga complexes obtained with the process according to the invention are useful as radiotracers and/or radiopharmaceuticals for the diagnosis and/or the treatment of specific diseases.

Therefore, the invention also relates to a process for preparing radiotracers and/or radiopharmaceuticals, i.e. molecules having affinity for a specific molecular receptor or substrates of an enzyme, labelled with the ⁶⁸Ga radioactive isotope. Radiotracers and/or radiopharmaceuticals are used for the diagnosis and/or the treatment of specific diseases, for example, through administration to a patient undergoing a diagnostic test. Such process includes at least one of the steps indicated above.

The invention also relates to a kit for the preparation of ⁶⁸Ga complexes comprising the necessary reagents for performing the complexation reaction into separate single-dose containers, and instructions for the operator who will perform the complexation reaction. The kit according to the invention can also be defined as a kit for the preparation of radiotracers and/or radiopharmaceuticals.

### BRIEF DESCRIPTION OF THE FIGURES

Figure 1 shows a paradigmatic UHPLC chromatogram (radiochemical detector) of a preparation of ⁶⁸Ga-DOTATOC obtained with the method according to the invention;
Figure 2 shows a paradigmatic UHPLC chromatogram (radiochemical detector) of a preparation of ⁶⁸Ga-DOTATOC obtained without the addition of ascorbic acid. Legend: 1: ⁶⁸Ga-DOTATOC 70%; 2: subproducts labelled with gallium-68 due to the oxidation of the precursor 28%; 3: ⁶⁸Ga(lll)-free 2%;
Figure 3 shows a paradigmatic UHPLC chromatogram (radiochemical detector) of a preparation of ⁶⁸Ga-DOTATOC obtained without gallium (III) salt. Legend: 1: ⁶⁸Ga-DOTATOC 91%; 3: ⁶⁸Ga(lll)-free 2%; 4: ⁶⁸Ga-subproducts (mixed complexes of hydrolysed gallium-68 or of the ML₂ type) 7%;
Figure 4 shows a paradigmatic UHPLC chromatogram (radiochemical detector) of a preparation of ⁶⁸Ga-DOTATOC, with high activity, without the addition of curcumin. Legend: 1: ⁶⁸Ga-DOTATOC 35%; 3: ⁶⁸Ga(lll)-free 3%; 2: ⁶⁸Ga-subproducts (subproducts due to the oxidation/radiolysis of the precursor) 62%;
Figure 5 shows a chromatogram of a preparation of ⁶⁸Ga-DOTATOC obtained under the same conditions as Figure 4, but with the presence of 2 µmol of curcumin in the reaction mixture. Legend: 1: ⁶⁸Ga-DOTATOC 98.7%; 3: ⁶⁸Ga(III)-free 0.8%; 4: ⁶⁸Ga-subproducts (mixed complexes of hydrolysed gallium-68 or of the ML₂ type) 0.5%.
Figure 6 shows a paradigmatic UHPLC chromatogram (radiochemical detector) of a preparation of ⁶⁸Ga-DOTATOC obtained using sodium ascorbate as a neutralising agent. Legend: 1: ⁶⁸Ga-DOTATOC 99.6%; 3: ⁶⁸Ga(III)-free 0.4%

### DETAILED DESCRIPTION OF THE INVENTION

The present invention relates to a process for the preparation of complexes of the ⁶⁸Ga radioisotope in which the complexation reaction between a chelator-functionalised molecule and ⁶⁸Ga occurs in the presence of a solution of a neutralising agent, ascorbic acid and/or a salt thereof, a salt of Ga³⁺ and/or curcumin or a derivative thereof.

According to the present invention, said neutralising agent is selected from an alkaline or alkaline-earth salt of formic acid or ascorbic acid and said derivative of curcumin is selected from diacetyl-curcumin (DAC) or bis(dehydroxy)curcumin (bDHC).

The process for the preparation of complexes of the ⁶⁸Ga radioisotope according to the present invention comprises a first step wherein an eluate containing ⁶⁸Ga is obtained by eluting a ⁶⁸Ge/⁶⁸Ga generator with a solution of an inorganic acid, for example hydrochloric acid.

The eluate containing ⁶⁸Ga is added directly, without purification, to a mixture comprising a chelator-functionalised molecule, a neutralising agent, ascorbic acid and/or a salt thereof, a salt of Ga³⁺and/or curcumin or a derivative thereof.

Preferably, the mixture thus obtained is stirred at ambient temperature or heated at a temperature preferably comprised between 90°C and 110°C, for a time comprised between 5 and 15 minutes.

At the end of heating, the pH of the mixture is preferably brought to a value comprised between 4.5 and 8.5, preferably between 5.5 and 6, through the addition of a stabilising solution. The stabilising solution is preferably a solution of a salt of ascorbic acid, for example, sodium ascorbate.

In one embodiment, in the process for the preparation of complexes of the ⁶⁸Ga radioisotope, the complexation reaction between a chelator-functionalised molecule and ⁶⁸Ga occurs in the presence of an alkaline or alkaline-earth salt of ascorbic acid (preferably sodium ascorbate) as the neutralising agent. In this case, curcumin and/or the gallium salt may be present in the reaction mixture or they may be absent. Furthermore, it is possible for ascorbic acid not to be added to the reaction mixture since it is formed, in part, following the dissociation of the ascorbic acid salt in solution.

The solution containing the ⁶⁸Ga complex obtained with both the embodiments of the process according to the invention, has a radiochemical purity (assessed through UHPLC) greater than 98±2%. The incorporation yield of ⁶⁸Ga by the chelator-functionalised molecule is > 98%.

Therefore, the ⁶⁸Ga complex obtained with the process according to the invention may be used for diagnostic or therapeutic purposes without a further purification treatment. In particular, the ⁶⁸Ga complex obtained with the process here described can be administered directly to the patient since the pH is comprised between 4.5 and 8.5 (suitable for human administration) and the radiochemical purity is very high. Furthermore, all the reagents used for the preparation (gallium salts, curcumin, ascorbic acid, neutralising solution and stabilising solution) are not toxic and are compatible with an *in vivo* administration.

In one embodiment, the process for preparing a ⁶⁸Ga complex comprises the steps of:
1) Preparing an eluate containing ⁶⁸Ga by eluting a ⁶⁸Ge/⁶⁸Ga generator with a solution of inorganic acid;
2) Adding the ⁶⁸Ga eluate, without a further purification, to a mixture containing a chelator-functionalised molecule, a neutralising agent, ascorbic acid and/or a salt thereof, a salt of Ga³⁺ and/or curcumin or a derivative thereof;
3) Heating or stirring at ambient temperature the mixture obtained in step 2);
4) At the end of the heating or stirring, bringing the pH of the mixture to a value comprised between 4.5 and 8.5 through the addition of a stabilising solution;
wherein said neutralising agent is selected from an alkaline or alkaline-earth salt of formic acid or ascorbic acid; said derivative of curcumin is selected from diacetyl-curcumin (DAC) or bis(dehydroxy)curcumin (bDHC); and said stabilising solution is a solution of an alkaline or alkaline-earth salt of ascorbic acid, preferably sodium ascorbate.

The eluate containing ⁶⁸Ga is obtained by eluting the generator with a strong inorganic acid, preferably hydrochloric acid.

The eluate thus obtained contains from 370 to 1850 MBq of ⁶⁸Ga.

The chelator-functionalised molecule is a molecule that shows affinity for a specific molecular receptor which, for example, is over-expressed by tumour cells or by cells affected by other diseases, or is a molecule that is involved in a specific physiological metabolism, or is a substrate of an enzyme. Examples of such molecules are: prostate specific membrane antigen (PSMA), useful for the determination of prostate cancer metastasis, somatostatin analogues, e.g. Tyr3-octreotide (TOC), tyr3-octreotate (TATE) and 1-Nal3-Octreotide (NOC), molecules having affinity for Vascular Endothelial Grow Factor VEGF receptors, bombesin analogues and molecules having affinity for Gastrin Releasing Peptide GRP receptors, molecules having affinity for estrogen receptors, molecules having affinity for integrin receptors (RDG α(V)β(3) and α(V)β(3) peptides), molecules involved in bone metabolism (diphosphonates), molecules having affinity for chemokine receptors (CXCR4).

The chelating agent that may be bonded, preferably through a covalent bond (e.g. through an amide bond), to the molecule having affinity for a receptor, is preferably chosen from: 1,4,7,10-tetraazacyclododecane-1,4,7,10-tetraacetic acid (DOTA), 1,4,7-triazacyclononane-1,4,7-trisacetic acid (NOTA), 3,6,9,15-tetraazabicyclo[9.3.1]pentadeca-1(15),11,13-triene-3,6,9-triacetic acid (PCTA), N,N'-Di(2-hydroxybenzyl)ethylenediamine-N,N'-diacetic acid (HBEDD), 6-[Bis(carboxymethyl)amino]-1,4-bis(carboxymethyl)-6-methyl-1,4-diazepane (AAZTA), tris-hydroxypyridinone (THP), 1,2-[[6-carboxypyridin-2-yl]methylamino]ethane) (DEDPA), 1,4,7-triazacyclononane phosphinic acid (TRAP), 2-[Bis[2-[bis(carboxymethyl)amino]ethyl]amino]acetic acid (DTPA), and derivatives thereof.

Derivatives of the aforementioned chelating agents are molecules that have the basic skeleton of the chelating agents with the addition of any other group that could be used for the connection with a biological molecule (e.g. amines, isothiocyanates, thioles, etc.) or another coordinating group (e.g. alkyl carboxyl groups, alcohols, alkyl-amines etc.), or other functional groups (e.g. alkyls, benzyls, etc.)

In general, all the chelating agents described in patent application EP2536691, are considered to be included within the scope of the invention.

The chelator-functionalised molecule used in the process according to the present invention can be obtained from the combination of any one of the chelating agents indicated above (and derivatives thereof) and any molecule having affinity for a specific molecular receptor, involved in a metabolic process or being a substrate of an enzyme such as, for example, those listed above.

Examples of chelator-functionalised molecules are: DOTA-TOC, DOTA-TATE, DOTA-NOC, DOTA-peptide, DOTA-PSMA e HBEDD-PSMA, DOTA-AMBA, DOTA-BPAMD (4-{[bis-(phosphonomethyl)) carbamoyl]methyl}-7,10-bis(carboxymethyl)-1,4,7,10-tetraazacyclododec-1-yl)acetic acid), Pentixafor.

The chelator-functionalised molecule is included in the mixture in quantities from 5 to 30 nmol.

The neutralising agent preferably used is a substance able to maintain the pH between 3 and 4.5, preferably between 3.3 and 3.9. Preferably, the neutralising agent is a salt of an alkaline or alkaline-earth metal of formic acid, for example sodium formate or potassium formate. Alternatively, the neutralising agent is a salt of an alkaline or alkaline-earth metal of ascorbic acid, for example sodium ascorbate or potassium ascorbate.

The neutralising agent is used to neutralise the excess of inorganic acid used for the elution of the generator. The neutralising agent must have a pKa of about 3.7, a low molecular weight and weak complexing power to be an optimal compound for the purposes of: i) reaching the best reaction pH; ii) preventing the precipitation of hydrolysed products of ⁶⁸Ga thanks to the transitory formation of ⁶⁸Ga-neutralising agent labile complexes; iii) facilitating the ligand exchange from the ⁶⁸Ga-neutralising agent complexes to the final ⁶⁸Ga-chelator-functionalised molecule complex. The quantity of neutralising agent used is the necessary quantity for obtaining the pH values indicated above. For example, the neutralising agent is present in the solution in a quantity comprised between 0.12 and 0.6 mmol.

Normally, the molar ratio between the chelator-functionalised molecule and ⁶⁸Ga³⁺ during the labelling reaction is about 4000: 1. Because of the high excess of chelating agent, the formation of ML₂ type complexes cannot be excluded. Furthermore, the partial hydrolysis of ⁶⁸Ga³⁺ could lead to the formation of mixed complexes of the Ga(OH)(chelating) type. The presence of small quantities of a gallium (III) salt promotes the formation of metal complexes with a metal:chelating agent ratio of 1:1. On the other hand, the quantity of gallium (III) added to the reaction must be carefully assessed since gallium (III) competes with Gallium-68 in the formation of the complex with the chelating agent.

It has been observed experimentally (see the "experimental tests" section), through comparison experiments, that the absence of Ga (III) salts in the mixture causes the formation of a high quantity of subproducts, for example, mixed complexes of hydrolysed Ga-68 or of the ML₂ type.

A gallium (III) salt preferably used in the mixture is a gallium chloride or nitrate, preferably in quantities from 50 to 150 pmol.

The quantity of ascorbic acid and/or a salt thereof is comprised between 2 and 10 µmol.

Ascorbic acid is a weak reducing agent and strong antioxidising agent. The presence of small quantities of ascorbic acid prevents the oxidation of the chelator-functionalised molecule (often such molecules contain amino acids or other groups prone to oxidation at high temperature). The advantages of the use of ascorbic acid are highlighted by the experiments included in the "experimental tests" section.

Curcumin is a natural molecule extracted from the curcuma longa root. It has extremely high antioxidising and radical scavenging properties (higher than ascorbic acid) thanks to the various tautomeric forms stabilised by resonance. Its presence in the formulation guarantees the stability of the precursors also at very high levels of activity such as those emitted by a 50 mCi generator at the beginning of its use. The presence of a di-ketone group in equilibrium with the keto-enolic form also provides curcumin with good complexing properties in relation to Fe³⁺, Cu²⁺ and Zn²⁺ (which are the main cations competing in the complexation reaction of the precursor with gallium-68). On the other hand, curcumin does not form Ga(lll) complexes in the pH interval of the reaction but mainly at pH 5, therefore it can be considered to be a selective sequestering agent in relation to some metal contaminants. Finally, curcumin has a practically negligible toxicity while its beneficial effects are known, such as being anti-inflammatory, anti-tumour and protective for the central nervous system.

The advantages of the use of curcumin or its derivatives in the mixture according to the invention are highlighted in the experiments performed and reported in the "experimental tests" section.

The derivatives of curcumin which can be used in the process according to the invention are diacetyl-curcumin (DAC) and bis(dehydroxy)curcumin (bDHC). The quantity of curcumin and/or a derivative thereof is preferably comprised between 0.2 and 5 µmol, preferably between 0.2 and 2 µmol.

In one embodiment the gallium (III) salt and curcumin (and/or a derivative thereof) are present simultaneously in the reaction mixture.

The reaction mixture is stirred at ambient temperature for a time comprised between 5 and 15 minutes or heated at a temperature comprised between 80 and 120°C, preferably between 90 and 110°C. The heating time is preferably comprised between 5 and 15 minutes.

The heating can be performed with conventional means, for example through a heating thermoblock or through the use of microwaves.

The stabilising solution used to bring the pH to a value between 4.5 and 8.5 is preferably an ascorbate solution of an alkaline or alkaline-earth metal. For example, the solution may be a sodium ascorbate solution.

At the end of the reaction, the pH of the solution is between 3 and 4, therefore largely outside the range at which it can be injected to human beings (4.5-8.5). The ascorbate solution has both the function of bringing the pH of the preparation into the range at which it can be injected (preferably about 5.5) and of protecting the product from auto-radiolysis.

In one embodiment, in the process for the preparation of complexes of the radioisotope ⁶⁸Ga, the complexation reaction between a chelator-functionalised molecule and ⁶⁸Ga occurs in the presence of an alkaline or alkaline-earth salt of ascorbic acid (preferably sodium ascorbate) as the neutralising agent. In this case, curcumin and/or the gallium salt may be present in the reaction mixture or they may be absent. Furthermore, it is possible for ascorbic acid not to be added to the reaction mixture since it is formed, in part, following the dissociation of the ascorbic acid salt in solution.

In other words, in step 2) of the process for the preparation of complexes of the ⁶⁸Ga radioisotope, the eluate of ⁶⁸Ga, without further purification, is added to a mixture containing a chelator-functionalised molecule and, as a neutralising agent, an alkaline or alkaline-earth salt of ascorbic acid, optionally in presence of a salt of Ga³⁺ and/or curcumin or a derivative thereof.

Steps 3) and 4) of the process remain unchanged.

The present invention also relates to a kit for the preparation of a ⁶⁸Ga complex comprising the necessary reagents for performing the complexation reaction into separate single-dose containers, and instructions for the operator who will perform the complexation reaction.

In particular, the kit according to the invention comprises a series of separate containers each containing one or more of the necessary components for the application of the method according to the invention. Preferably, the kit according to the invention comprises:
- a vial containing a chelator-functionalised molecule, a neutralising agent, ascorbic acid and/or a salt thereof, a salt of Ga³⁺ and/or curcumin or derivatives thereof;
- a vial or a syringe containing a stabilising solution, preferably a solution of a salt of ascorbic acid;
- optionally, a vial or a syringe containing a solution of inorganic acid.

In an alternative embodiment, the kit according to the invention comprises:
- a vial containing a chelator-functionalised molecule;
- a vial or syringe containing a neutralising agent, ascorbic acid and/or a salt thereof, a salt of Ga³⁺ and/or curcumin or derivatives thereof;
- a vial or a syringe containing a stabilising solution, preferably a solution of a salt of ascorbic acid;
- optionally, a vial or a syringe containing a solution of inorganic acid.

The ingredients indicated above are as defined hereinabove. In particular, the neutralising agent is chosen from an alkaline or alkaline-earth salt of ascorbic acid or formic acid. For example, the neutralising agent may be sodium or potassium ascorbate, or sodium or potassium formate.

In one embodiment, the kit for preparing a ⁶⁸Ga complex comprises:
- a vial containing a chelator-functionalised molecule, an alkaline or alkaline-earth salt of ascorbic acid and optionally a salt of Ga³⁺ and/or curcumin or derivatives thereof;
- a vial or a syringe containing a stabilising solution, preferably a solution of a salt of ascorbic acid;
- optionally, a vial or a syringe containing a solution of inorganic acid.

In one embodiment, the kit for preparing a ⁶⁸Ga complex comprises:
- a vial containing a chelator-functionalised molecule and an alkaline or alkaline-earth salt of ascorbic acid;
- optionally a vial containing a salt of Ga³⁺ and/or curcumin or derivatives thereof;
- a vial or a syringe containing a stabilising solution, preferably a solution of a salt of ascorbic acid;
- optionally, a vial or a syringe containing a solution of inorganic acid.

Preferably, the components are inserted into sealed containers as indicated above which are then packaged together with instructions for performing the method according to the invention.

The kit according to the invention can also be used as a part of an automatic system or a remotely controlled mechanical system that automatically performs the elution of the gallium-68 generator and/or the subsequent heating. In this formulation the vial containing the chelator-functionalised molecule is directly connected to the elution system and/or to the heating system of the automatic system or the content of this vial is transferred into the reactor of the automatic system before or during synthesis. In the same way, the vial containing the stabilising solution can be connected to the automatic system and its contents may be transferred into the reaction vial or into the vial of the final product.

### EXPERIMENTAL TESTS

### Materials and Methods

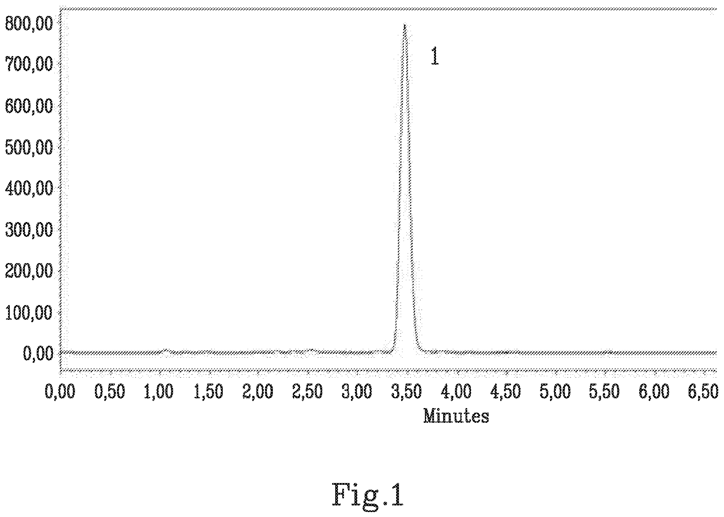

The ⁶⁸Ge/⁶⁸Ga generators were eluted manually with 4 mL of an HCI solution (0.1N or 0.05N according to whether the IGG 100 or itG generator was used, respectively). The eluates, containing about 555 MBq of Gallium-68, were entirely collected in a disposable vial containing 7-20 nmol of precursors (chelator-functionalised molecules; the DOTATOC precursor was used as a representative of this class of compounds), 5-6 µmol of ascorbic acid, 0.2-2 µmol of curcumin, 50-100 pmol of Ga (III) (as chloride or nitrate) and the appropriate quantity of sodium formate in order to maintain the reaction pH between 3.3-3.8. The mixture was then heated at 100°C for 15 minutes and then diluted manually with 6 mL of a 1 M sodium ascorbate solution contained in the formulation syringe. Aliquots of the mixture were collected at 10, 15 and 20 minutes to assess the progression of the reaction through UHPLC analysis. The kit prototypes were prepared in a sterile environment by opening the vials, adding the reagents in solid form and re-sealing the vials again with new septa. Each preparation was performed at least in triplicate and all the incorporation yields were calculated considering the radiochemical purity (RCP), obtained from the UHPLC analyses. No further purification was performed on the preparations. A paradigmatic UHPLC chromatogram (radiochemical detector) of a preparation of ⁶⁸Ga-DOTATOC is shown in Figure 1. From the chromatogram in Fig. 1 it can be deduced that the method according to the invention allows to obtain a molecule labelled with ⁶⁸Ga with a degree of purity of about 98±2% and with a method that does not require any final purification step for obtaining such purity percentage.

### Comparative experiments

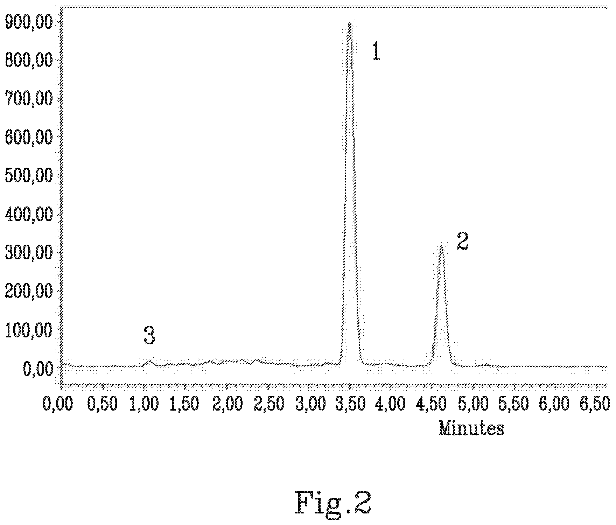

To underline the advantages obtained with the addition of ascorbic acid to the mixture according to the invention, a series of experiments analogous to those described above was performed (the DOTATOC precursor was used as a representative) but without the addition of ascorbic acid to the mixture. The incorporation yield and the radiochemical purity were compared to those previously obtained. A paradigmatic UHPLC chromatogram (radiochemical detector) of a preparation of ⁶⁸Ga-DOTATOC without the addition of ascorbic acid is shown in Figure 2. As it can be deduced from the chromatogram in figure 2, the lack of ascorbic acid determines the formation of impurities in terms of subproducts labelled with gallium-68 due to the oxidation of the precursor and of ⁶⁸Ga(lll)-free.

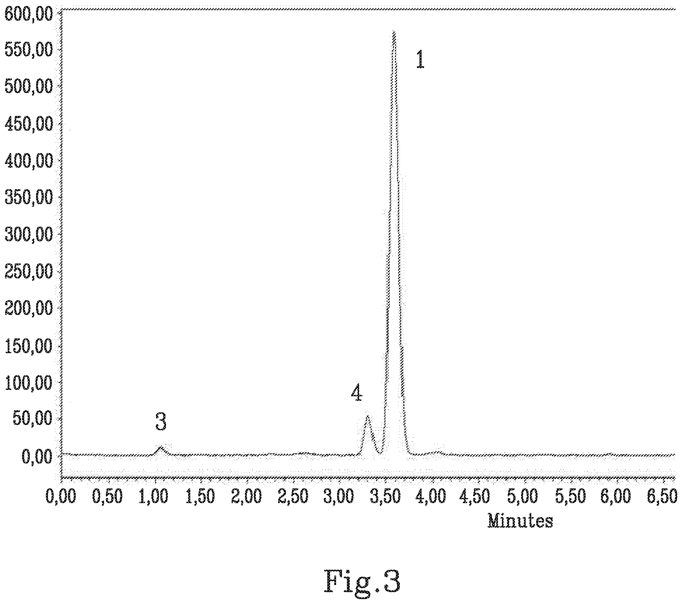

In order to underline the advantages obtained with the addition of a salt of gallium (III) in the preparations according to the invention, a series of experiments analogous to those previously described was performed, but without the addition of the gallium (III) salt to the reaction mixture. The incorporation yield and the radiochemical purity were compared to the preparations previously obtained. A paradigmatic UHPLC chromatogram (radiochemical detector) of a preparation of ⁶⁸Ga-DOTATOC without the gallium (III) salt is shown in Figure 3.

From the chromatogram shown in figure 3 it can be deduced that the lack of Ga(lll) salts leads to the formation of subproducts due to the formation of mixed complexes of hydrolysed gallium-68 or of the ML₂ type and also to free gallium (III).

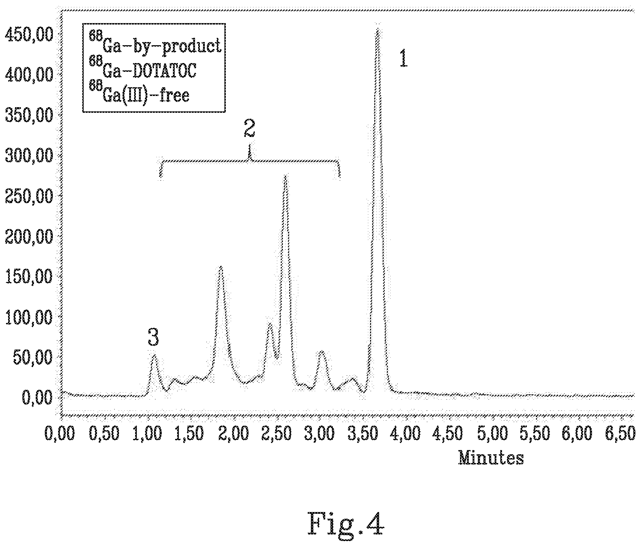

To underline the advantages obtained with the addition of curcumin in the preparations according to the invention, a series of experiments analogous to those described above was performed but under particular oxidative stress conditions (e.g. at high levels of radioactivity or in an oxygen-rich environment) (the DOTATOC precursor was used as a representative), but without the addition of curcumin to the mixture. The incorporation yield and the radiochemical purity were compared to those previously obtained. A paradigmatic UHPLC chromatogram (radiochemical detector) of a preparation of 68Ga-DOTATOC without the addition of curcumin is shown in Figure 4.

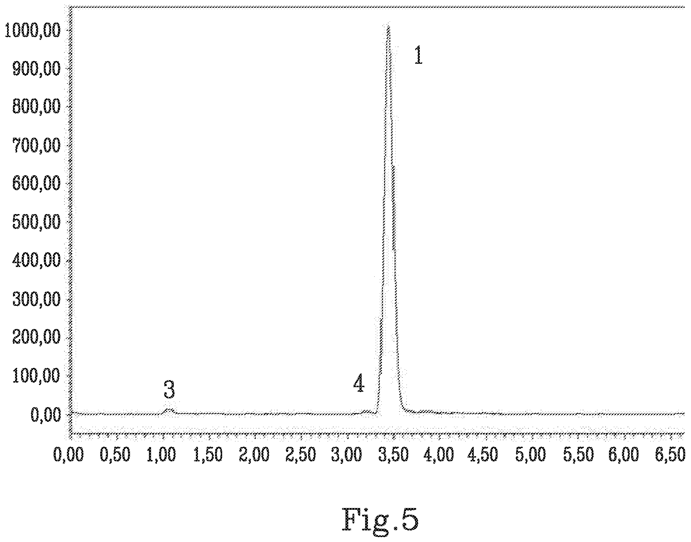

In the absence of curcumin a net worsening of the reaction purity is observed, with a quantity of about 62% of subproducts due to the oxidation/radiolysis of the precursor and about 3% of free gallium (III). Under the same conditions, the presence of 2 µmol of curcumin in the reaction mixture kept the radiochemical purity, and therefore the reaction yield, at suitable levels, as shown in Figure 5.

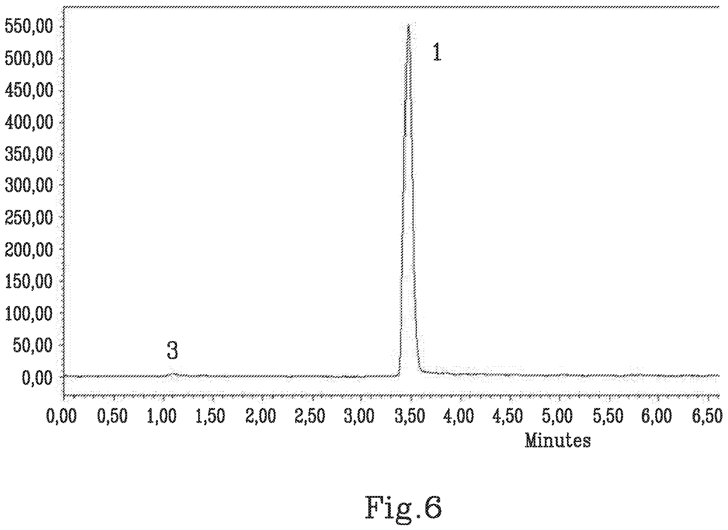

To underline the advantages obtained from a reaction performed using sodium ascorbate as a neutralising agent, a series of experiments analogous to those described above was performed, but without the addition of gallium (III) salt, ascorbic acid and curcumin to the reaction mixture and in which sodium formate was substituted by an equivalent quantity of sodium ascorbate. As shown in figure 6, the purity of the final compound is maintained > 98 % using these conditions.

### Quality controls

Quality controls were performed on each preparation following the method prescribed in the European pharmacopoeia for injectable preparations of ⁶⁸Ga-DOTATOC. The UHPLC analyses were performed at a flow rate of 0.35 mL/min with a mobile phase consisting of a mixture of 78% acetonitrile (ACN) and 22% of a 0.1% v/v solution of trifluoroacetic acid/water. The wavelength of the UV detector was set to 220 nm and the temperature of the column was set at 30°C. In order to identify the chromatographic peaks during the analysis, preparations of hydrolysed products of gallium-68 and of ⁶⁸Ga³⁺-free were used as reference standards. The preparations were obtained as indicated in literature (Asti M, Lori M, Capponi PC et al. Influence of different chelators on the radiochemical properties of a 68-Gallium labelled bombesin analogue. Nucl Med Biol. 2014;41 (1):24-35). A calibration curve was calculated using reference solutions at different concentrations of TOC, DOTATOC and natGa-DOTATOC. These solutions were also used to determine the retention time of the free ligand and of the resolution product thereof (TOC). Sterility (fungi, bacterial culture in aerobiosis and anaerobiosis) and absence of bacterial endotoxins (LAL test) were tested in all the samples according to the European pharmacopoeia standards. The quantity of germanium-68 was quantified with radiometric methods (gamma spectrometry) after 10 days of decay.

### Results

The radio-labelling of chelator-functionalised molecules (⁶⁸Ga-DOTATOC for example) with gallium-68 using the method according to the invention, leads to an incorporation yield > 98% when suitable conditions are used (pH, heating, etc.). These results have confirmed the suitability of a kit-based approach and the feasibility of preparing such kits, pre-filled with the reagents used. The radiochemical purity of the preparations (assessed through UHPLC) was always > 98±2%. By applying the UHPLC conditions described herein, the following retention times are observed: ⁶⁸Ga³⁺ free = 1.1 minutes, ⁶⁸Ga-hydrolysed products = 1.3 minutes, ⁶⁸Ga-subproducts = 2.6, 3.3, 4.1 minutes and ⁶⁸Ga-DOTATOC = 3.6 min.

It has been shown that a UHPLC instrument guarantees a more reliable analysis and a higher peak separation. Therefore, the simple use of this instrument rather than a normal HPLC for determining the RCP guarantees more accurate quality controls on the final product. For example, with a HPLC system, the separation of ⁶⁸Ga-DOTATOC from subproduct no. 4 is difficult to obtain. On the other hand, TLC analysis has always shown a RCP > 99% in all the tests we have performed, regardless of the addition of ascorbic acid, Gallium (III) salts or curcumin. This underlines the fact that the method used for the analyses is fundamental for assessing the reliability of the formulation. Other processes known in the field used analysis methods with lower separating capacity and can therefore have underestimated the presence of any impurities in the final product.

Despite the absence of a further purification of the eluates, the loss of germanium-68 contained in the final vial was in the order of 10⁻⁴ and 10⁻⁵% according to the generator used. Each preparation was sterile. The level of bacterial endotoxins of the kit preparations is very low (about 4 UI/mL for both generators).

### Example 1

### Labelling of ⁶⁸Ga-PSMA with 1.1 ml of an eluate of 0.1 M HCl:

A 30 mCi commercial generator (IRE-Elit) is eluted with 1.1 ml of ultrapure 0.1 M HCI directly in a vial containing 20 ug of DOTA-PSMA precursor, 1 mg of ascorbic acid and 10 mg of sodium formate. The vial is heated at 95°C for 15 min and then diluted with 5 ml of 1.5 M sodium ascorbate solution. The radiochemical purity of the product, assessed by reverse-phase UHPLC, is > 98%.

### Example 2

### Labelling of ⁶⁸Ga-DOTA-peptide with 4 ml of an eluate of 0.1 M HCl:

A 50 mCi commercial generator (Eckert & Ziegler) with a stationary phase of TiO₂ is eluted with 4ml of ultrapure 0.1 M HCl directly in a vial. An aqueous solution containing: 30 ug of DOTA-peptide precursor, 1 mg of ascorbic acid, 0.5 mg of curcumin, 13 ng of GaCl3 and 40 mg of sodium formate is added to the vial. The vial is heated at 95°C for 15 min and then diluted with 5 ml of 1.5 M sodium ascorbate solution. The radiochemical purity of the product, assessed by reverse-phase UHPLC, is > 98%.

### Example 3

### Labelling of ⁶⁸Ga-DOTA-peptide with 4 ml of an eluate of 0.05 M HCI:

A 30 mCi commercial generator (ITG) with a stationary organic phase is eluted with 4 ml of ultrapure 0.05 M HCI directly into a vial containing 50 ug of the DOTA-peptide precursor. An aqueous solution containing: 1 mg of ascorbic acid, 19 ng of Ga (NO₃)₃ and 19 mg of sodium formate is added to the vial. The vial is heated at 95°C for 15 min and then diluted with 5 ml of 1.5 M sodium ascorbate solution. The radiochemical purity of the product, assessed by reverse-phase UHPLC, is > 98%.

### Example 4

### Labelling of ⁶⁸Ga-PSMA with 5 ml of an eluate of 0.1 M HCl

A 30 mCi commercial generator (Eckert & Ziegler) with a stationary phase of TiO₂ is eluted with 5 ml of ultrapure 0.1 M HCl directly in a vial containing 1 mg of ascorbic acid and 50 mg of sodium formate. 20 ug of HBED-PSMA precursor in aqueous solution are added to the vial. The vial is stirred and then diluted with 5 ml of 1.5 M sodium ascorbate solution. The radiochemical purity of the product, assessed by reverse-phase UHPLC, is > 98%.

### Example 5

### Labelling of ⁶⁸Ga-PSMA with 5 ml of an eluate of 0.05 M HCl

A 50 mCi commercial generator (ITG) with a stationary phase of organic material is eluted with 5 ml of ultrapure 0.05 M HCl directly in a vial containing 60 mg of sodium ascorbate + 25 µg of HBED-PSMA precursor. The vial is stirred and then diluted with 5 ml of 1.5 M sodium ascorbate solution. The radiochemical purity of the product, assessed by reverse-phase UHPLC, is > 98%.

## Claims

1. A process for preparing complexes of the ⁶⁸Ga radioisotope, wherein the complexation reaction between a chelator-functionalised molecule and ⁶⁸Ga occurs in the presence of a neutralising agent, ascorbic acid and/or salts thereof, a salt of Ga³⁺ and/or curcumin or a derivative thereof, wherein said neutralising agent is selected from an alkaline or alkaline-earth salt of formic acid or ascorbic acid and said derivative of curcumin is selected from diacetyl-curcumin (DAC) or bis(dehydroxy)curcumin (bDHC), wherein the process comprises a first step in which an eluate containing ⁶⁸Ga is obtained by eluting a ⁶⁸Ge/⁶⁸Ga generator with a solution of an inorganic acid and the eluate is added directly, without purification, to a mixture comprising the chelator-functionalised molecule, the neutralising agent, ascorbic acid and/or a salt thereof, the salt of Ga³⁺ and/or curcumin or a derivative thereof.

2. The process according to claim 1, wherein the inorganic acid is hydrochloric acid.

3. The process according to claim 1 or 2, wherein the mixture obtained after the first step is stirred at ambient temperature for a time comprised between 5-15 minutes or heated, preferably through the use of microwaves, at a temperature comprised between 80°C and 120°C for a time comprised between 5 and 15 minutes.

4. The process according to any of claims 1 to 3, wherein at the end of the complexation reaction the pH of the reaction mixture is brought to a value comprised between 4.5 and 8.5, preferably between 5.5 and 6, by adding a stabilising solution, wherein said stabilising solution is a solution of an alkaline or alkaline-earth salt of ascorbic acid, preferably sodium ascorbate.

5. A process for preparing complexes of the ⁶⁸Ga radioisotope, comprising the steps of:
1) Preparing an eluate containing ⁶⁸Ga by elution of a ⁶⁸Ge/⁶⁸Ga generator with an inorganic acid solution;
2) Adding the ⁶⁸Ga eluate, without further purification, to a mixture containing a chelator-functionalised molecule, a neutralising agent, ascorbic acid and/or a salt thereof, a salt of Ga³⁺ and/or curcumin or a derivative thereof;
3) Stirring at ambient temperature or heating the mixture obtained in step 2);
4) At the end of the heating, bringing the pH of the mixture to a value comprised between 4.5 and 8.5 by adding a stabilising solution;
wherein said neutralising agent is selected from an alkaline or alkaline-earth salt of formic acid or ascorbic acid; said derivative of curcumin is selected from diacetyl-curcumin (DAC) or bis(dehydroxy)curcumin (bDHC); and said stabilising solution is a solution of an alkaline or alkaline-earth salt of ascorbic acid, preferably sodium ascorbate.

6. The process according to claim 5, wherein the chelator-functionalised molecule is a molecule that shows affinity for a specific molecular receptor, preferably said molecule is selected from the group consisting of: prostate-specific membrane antigen (PSMA), somatostatin analogues, preferably Tyr3-octreotide (TOC), TATE and NOC, molecules having affinity for the VEGF receptors, bombesin analogues, molecules having affinity for the GRP receptors, molecules having affinity for the estrogen receptors, molecules having affinity for the integrin receptors (peptides RDG α(V)β(3) and α(V)β(3)), molecules involved in bone metabolism (diphosphonates), and molecules having affinity for the chemokine receptors (CXCR4), preferably said chelator-functionalised molecule is selected from: DOTA-TOC, DOTA-TATE, DOTA-NOC, DOTA-peptide, DOTA-PSMA and HBEDD-PSMA.

7. The process according to claim 5 or 6, wherein the chelator that functionalises the molecule having affinity for a receptor, is selected from the group consisting of: 1,4,7,10-tetraazacyclododecane-1,4,7,10-tetraacetic acid (DOTA), 1,4,7-triazacyclononane-1,4,7-triacetic acid (NOTA), 3,6,9,15-tetraazabicyclo[9.3.1]pentadeca-1(15),11,13-triene-3,6,9-triacetic acid (PCTA), N,N'-Di(2-hydroxybenzyl)ethylenediamine-N,N'-diacetic acid (HBEDD), 6-[Bis(carboxymethyl)amino]-1,4-bis(carboxymethyl)-6-methyl-1,4-diazepane (AAZTA), tris-hydroxypyridinone (THP), 1,2-[[6-carboxypyridin-2-yl]methylamino]ethane) (DEDPA), 1,4,7-triazacyclononane phosphinic acid (TRAP), 2-[Bis[2-[bis(carboxymethyl)amino]ethyl]amino]acetic acid (DTPA), and derivates thereof.

8. The process according to any of claims 1 to 7, wherein said neutralising agent is a sodium or potassium salt of formic acid, or a sodium or potassium salt of ascorbic acid, wherein, preferably, when the neutralising agent is an alkaline or alkaline-earth salt of ascorbic acid, gallium salt and curcumin are not present in the solution.

9. A kit for preparing a ⁶⁸Ga complex comprising the reagents necessary for carrying out the complexation reaction according to any of claims 1 to 8, into separate single-dose containers, and instructions for the operator who will perform the complexation reaction.

10. The kit according to claim 9, comprising:
- a vial containing a chelator-functionalised molecule, a neutralising agent, ascorbic acid and/or a salt thereof, a salt of Ga³⁺ and/or curcumin or derivatives thereof;
- a vial or a syringe containing a stabilising solution;
- optionally, a vial or a syringe containing a solution of an inorganic acid.

11. The kit according to claim 9 comprising:
- a vial containing a chelator-functionalised molecule;
- a vial or syringe containing a neutralising agent, ascorbic acid and/or a salt thereof, a salt of Ga3+ and/or curcumin or derivatives thereof;
- a vial or a syringe containing a stabilising solution;
- optionally, a vial or a syringe containing a solution of inorganic acid.

12. The kit according to claim 9 comprising:
- a vial containing a chelator-functionalised molecule, an alkaline or alkaline-earth salt of ascorbic acid and optionally a salt of Ga³⁺ and/or curcumin or derivatives thereof;
- a vial or a syringe containing a stabilising solution;
- optionally, a vial or a syringe containing a solution of inorganic acid.

13. The kit according to claim 9 comprising:
- a vial containing a chelator-functionalised molecule and an alkaline or alkaline-earth salt of ascorbic acid;
- optionally a vial containing a salt of Ga³⁺ and/or curcumin or derivatives thereof;
- a vial or a syringe containing a stabilising solution;
- optionally, a vial or a syringe containing a solution of inorganic acid.

14. Use of the kit according to claims 9 to 13 in an automatic synthesis module.

## Patentansprüche

1. Verfahren zur Herstellung von Komplexen des ⁶⁸Ga― Radioisotops, wobei die Komplexierungsreaktion zwischen einem Chelatbildner-funktionalisierten Molekül und ⁶⁸Ga in Gegenwart eines Neutralisationsmittels, Ascorbinsäure und/oder Salzen davon, eines Salzes von Ga³⁺ und/oder Curcumin oder eines Derivats davon stattfindet, wobei das Neutralisationsmittel aus einem Alkali- oder Erdalkalisalz von Ameisensäure oder Ascorbinsäure ausgewählt ist und das Curcuminderivat aus Diacetylcurcumin (DAC) oder Bis(dehydroxy)curcumin (bDHC) ausgewählt ist, wobei das Verfahren einen ersten Schritt umfasst, in dem ein ⁶⁸Ga enthaltendes Eluat durch Eluieren eines ⁶⁸Ge/⁶⁸Ga-Generators mit einer Lösung einer anorganischen Säure erhalten wird und das Eluat ohne Reinigung direkt zu einer Mischung zugegeben wird, die das Chelatbildner-funktionalisierte Molekül, das Neutralisationsmittel, Ascorbinsäure und/oder ein Salz davon, das Salz von Ga³⁺ und/oder Curcumin oder ein Derivat davon umfasst.

2. Verfahren nach Anspruch 1, wobei die anorganische Säure Salzsäure ist.

3. Verfahren nach Anspruch 1 oder 2, wobei die nach dem ersten Schritt erhaltene Mischung bei Umgebungstemperatur für eine Zeit zwischen 5-15 Minuten gerührt oder, vorzugsweise unter Verwendung von Mikrowellen, auf eine Temperatur zwischen 80 °C und 120 °C für eine Zeit zwischen 5 und 15 Minuten erhitzt wird.

4. Verfahren nach einem der Ansprüche 1 bis 3, wobei am Ende der Komplexierungsreaktion der pH-Wert der Reaktionsmischung durch Zugabe einer Stabilisierungslösung auf einen Wert zwischen 4,5 und 8,5, vorzugsweise zwischen 5,5 und 6, gebracht wird, wobei die Stabilisierungslösung eine Lösung eines Alkali- oder Erdalkalisalzes der Ascorbinsäure, vorzugsweise Natriumascorbat ist.

5. Verfahren zur Herstellung von Komplexen des ⁶⁸Ga― Radioisotops, umfassend die Schritte:
1) Herstellen eines ⁶⁸Ga enthaltenden Eluats durch Elution eines ⁶⁸Ge/⁶⁸Ga-Generators mit einer anorganischen Säurelösung;
2) Zugeben des ⁶⁸Ga-Eluats ohne weitere Reinigung zu einer Mischung, die ein Chelatbildnerfunktionalisiertes Molekül, ein Neutralisationsmittel, Ascorbinsäure und/oder ein Salz davon, ein Salz von Ga³⁺ und/oder Curcumin oder ein Derivat davon enthält;
3) Rühren bei Umgebungstemperatur oder Erhitzen der in Schritt 2 erhaltenen Mischung;
4) Am Ende des Erhitzens Bringen des pH-Werts der Mischung auf einen Wert zwischen 4,5 und 8,5 durch Zugabe einer Stabilisierungslösung;
wobei das Neutralisationsmittel aus einem Alkali- oder Erdalkalisalz von Ameisensäure oder Ascorbinsäure ausgewählt ist; das Curcumin-Derivat ist aus Diacetyl-Curcumin (DAC) oder Bis(dehydroxy)curcumin (bDHC) ausgewählt; und die Stabilisierungslösung ist eine Lösung eines Alkali- oder Erdalkalisalzes von Ascorbinsäure, vorzugsweise Natriumascorbat.

6. Verfahren nach Anspruch 5, wobei das Chelatbildner-funktionalisierte Molekül ein Molekül ist, das Affinität zu einem spezifischen molekularen Rezeptor zeigt, vorzugsweise ist das Molekül ausgewählt aus der Gruppe bestehend aus: Prostata-spezifischem Membranantigen (PSMA), Somatostatin-Analoga, vorzugsweise Tyr3- Octreotid (TOC), TATE und NOC, Molekülen mit Affinität zu den VEGF-Rezeptoren, Bombesin-Analoga, Molekülen mit Affinität zu den GRP-Rezeptoren, Molekülen mit Affinität zu den Östrogenrezeptoren, Molekülen mit Affinität zu den Integrinrezeptoren (Peptide RDG α(V) β(3) und α(V)β(3)), am Knochenstoffwechsel beteiligten Molekülen (Diphosphonate) und Molekülen mit Affinität zu den Chemokinrezeptoren (CXCR4), vorzugsweise ist das Chelatbildner-funktionalisierte Molekül ausgewählt aus: DOTA-TOC, DOTA-TATE, DOTA-NOC, DOTA-Peptid, DOTA-PSMA und HBEDD-PSMA.

7. Verfahren nach Anspruch 5 oder 6, wobei der Chelatbildner, der das Molekül mit Affinität zu einem Rezeptor funktionalisiert, ausgewählt ist aus der Gruppe bestehend aus: 1,4,7,10-Tetraazacyclododecan-1,4,7,10-Tetraessigsäure (DOTA), 1,4,7-Triazacyclononan-1,4,7- Triessigsäure (NOTA), 3,6,9, 15-Tetraazabicyclo[9.3.1]pentadeca-1(15),11,13-trien-3,6,9-triessigsäure (PCTA), N,N'-Di(2-hydroxybenzyl)ethylendiamin-N,N'-diessigsäure (HBEDD), 6-[Bis(carboxymethyl)amino]-1,4-bis(carboxymethyl)-6-methyl-1,4-diazepan (AAZTA), Tris-hydroxypyridinon (THP), 1,2 -[[6-carboxypyridin-2-yl]methylamino]ethan) (DEDPA), 1,4,7-Triazacyclononanphosphinsäure (TRAP), 2-[Bis[2-[bis(carboxymethyl)amino]ethyl]amino]essigsäure (DTPA) und Derivate davon.

8. Verfahren nach einem der Ansprüche 1 bis 7, wobei das Neutralisationsmittel ein Natrium- oder Kaliumsalz von Ameisensäure oder ein Natrium- oder Kaliumsalz von Ascorbinsäure ist, wobei vorzugsweise Galliumsalz und Curcumin in der Lösung nicht vorhanden sind, wenn das Neutralisationsmittel ein Alkali- oder Erdalkalisalz der Ascorbinsäure ist.

9. Kit zur Herstellung eines ⁶⁸Ga-Komplexes, umfassend die Reagenzien, die zur Durchführung der Komplexierungsreaktion nach einem der Ansprüche 1 bis 8, in separaten Einzeldosisbehältern, erforderlich sind und Anweisungen für den Bediener, der die Komplexierungsreaktion durchführt.

10. Kit nach Anspruch 9, umfassend:
- eine Durchstechflasche, die ein Chelatbildnerfunktionalisiertes Molekül, ein Neutralisationsmittel, Ascorbinsäure und/oder ein Salz davon, ein Salz von Ga3+ und/oder Curcumin oder Derivate davon enthält;
- eine Durchstechflasche oder eine Spritze, die eine Stabilisierungslösung enthalten;
- wahlweise eine Durchstechflasche oder eine Spritze, die eine Lösung einer anorganischen Säure enthalten.

11. Kit nach Anspruch 9, umfassend:
- eine Durchstechflasche, die ein Chelatbildnerfunktionalisiertes Molekül enthält;
- eine Durchstechflasche oder eine Spritze, die ein Neutralisationsmittel, Ascorbinsäure und/oder ein Salz davon, ein Salz von Ga3+ und/oder Curcumin oder Derivate davon enthalten;
- eine Durchstechflasche oder eine Spritze, die eine Stabilisierungslösung enthalten; - wahlweise eine Durchstechflasche oder eine Spritze, die eine Lösung einer anorganischen Säure enthalten.

12. Kit nach Anspruch 9, umfassend:
- eine Durchstechflasche, die ein Chelatbildnerfunktionalisiertes Molekül, ein Alkali- oder Erdalkalisalz von Ascorbinsäure und wahlweise ein Salz von Ga³⁺ und/oder Curcumin oder Derivate davon enthält;
- eine Durchstechflasche oder eine Spritze, die eine Stabilisierungslösung enthalten;
- wahlweise eine Durchstechflasche oder eine Spritze, die eine anorganische Säure enthalten.

13. Kit nach Anspruch 9, umfassend:
- eine Durchstechflasche, die ein Chelatbildnerfunktionalisiertes Molekül und ein Alkali- oder Erdalkalisalz der Ascorbinsäure enthält;
- wahlweise eine Durchstechflasche, die ein Salz von Ga³⁺ und/oder Curcumin oder Derivate davon enthält;
- eine Durchstechflasche oder eine Spritze, die eine Stabilisierungslösung enthalten;
- wahlweise eine Durchstechflasche oder eine Spritze, die eine anorganische Säure enthalten.

14. Verwendung des Kits nach den Ansprüchen 9 bis 13 in einem automatischen Synthesemodul.

## Revendications

1. Procédé de préparation de complexes du radio-isotope ⁶⁸Ga, dans lequel la réaction de complexation entre une molécule fonctionnalisée par un chélateur et le ⁶⁸Ga se produit en présence d'un agent neutralisant, d'un acide ascorbique et/ou de ses sels, d'un sel de Ga³⁺ et/ou de curcumine ou d'un dérivé de celle-ci, dans lequel ledit agent neutralisant est choisi parmi un sel alcalin ou alcalino-terreux d'acide formique ou d'acide ascorbique et ledit dérivé de curcumine est choisi parmi le diacétyle-curcumine (DAC) ou la bis(déhydroxy)curcumine (bDHC), dans lequel le procédé comprend une première étape dans laquelle un éluat contenant du ⁶⁸Ga est obtenu par élution d'un générateur de ⁶⁸Ge/⁶⁸Ga avec une solution d'un acide inorganique et l'éluat est ajouté directement, sans purification, à un mélange comprenant la molécule fonctionnalisée par un chélateur, l'agent neutralisant, l'acide ascorbique et/ou un sel de celui-ci, le sel de Ga³⁺ et/ou la curcumine ou un dérivé de celle-ci.

2. Procédé selon la revendication 1, dans lequel l'acide inorganique est de l'acide chlorhydrique.

3. Procédé selon la revendication 1 ou 2, dans lequel le mélange obtenu après la première étape est agité à température ambiante pendant une durée comprise entre 5 et 15 minutes ou chauffé, de préférence par l'utilisation de microondes, à une température comprise entre 80 et 120 °C pendant une durée comprise entre 5 et 15 minutes.

4. Procédé selon l'une quelconque des revendications 1 à 3, dans lequel, à la fin de la réaction de complexation, le pH du mélange réactionnel est amené à une valeur comprise entre 4,5 et 8,5, de préférence entre 5,5 et 6, en ajoutant une solution stabilisante, dans lequel ladite solution stabilisante étant une solution d'un sel alcalin ou alcalino-terreux d'acide ascorbique, de préférence d'ascorbate de sodium.

5. Procédé de préparation de complexes du radio-isotope ⁶⁸Ga, comprenant les étapes de :
1) Préparer un éluat contenant du ⁶⁸Ga par élution d'un générateur de ⁶⁸Ge/⁶⁸Ga avec une solution d'acide inorganique ;
2) Ajouter l'éluat de ⁶⁸Ga, sans purification supplémentaire, à un mélange contenant une molécule fonctionnalisée par un chélateur, un agent neutralisant, de l'acide ascorbique et/ou un sel de celui-ci, un sel de Ga³⁺ et/ou de la curcumine ou un dérivé de celle-ci ;
3) Agiter à température ambiante ou chauffer le mélange obtenu à l'étape 2) ;
4) À la fin du chauffage, amener le pH du mélange à une valeur comprise entre 4,5 et 8,5 en ajoutant une solution stabilisante ;
dans lequel ledit agent neutralisant est choisi parmi un sel alcalin ou alcalino-terreux d'acide formique ou d'acide ascorbique ; ledit dérivé de curcumine est choisi parmi le diacétyle-curcumine (DAC) ou la bis(déhydroxy)curcumine (bDHC) ; et ladite solution stabilisante est une solution d'un sel alcalin ou alcalino-terreux d'acide ascorbique, de préférence d'ascorbate de sodium.

6. Procédé selon la revendication 5, dans lequel la molécule fonctionnalisée par un chélateur est une molécule présentant une affinité pour un récepteur moléculaire spécifique, de préférence ladite molécule est choisie dans le groupe constitué par : l'antigène membranaire spécifique de la prostate (PSMA), les analogues de la somatostatine, de préférence le Tyr3-octréotide (TOC), le TATE et le NOC, les molécules ayant une affinité pour les récepteurs du FCEV, les analogues de la bombésine, les molécules ayant une affinité pour les récepteurs GRP, les molécules ayant une affinité pour les récepteurs des œstrogènes, les molécules ayant une affinité pour les récepteurs des intégrines (peptides RDG α(V)β(3) et α(V)β(3)), les molécules impliquées dans le métabolisme osseux (diphosphonates), et les molécules ayant une affinité pour les récepteurs des chimiokines (CXCR4), de préférence ladite molécule fonctionnalisée par un chélateur est choisie parmi : DOTA-TOC, DOTA-TATE, DOTA-NOC, DOTA-peptide, DOTA-PSMA et HBEDD-PSMA.

7. Procédé selon la revendication 5 ou 6, dans lequel le chélateur qui fonctionnalise la molécule ayant une affinité pour un récepteur, est choisi dans le groupe constitué par : l'acide 1,4,7,10-tétraazacyclododécane-1,4,7,10-tétraacétique (DOTA), l'acide 1,4,7-triazacyclononane-1,4,7- triacétique (NOTA), l'acide 3,6,9, 15-tétraazabicyclo[9.3. 1 ]pentadéca-1(15),11,13-triène-3,6,9-triacétique (PCTA), l'acide N,N'-Di(2- hydroxybenzyl)éthylènediamine-N,N'-diacétique (HBEDD), 6-[Bis(carboxyméthyl)amino]-1,4-bis(carboxyméthyl)-6-méthyl-1,4- diazépane (AAZTA), tris-hydroxypyridinone (THP), 1,2-[[6-carboxypyridin-2-yl]méthylamino]éthane) (DEDPA), l'acide 1,4,7-triazacyclononane phosphinique (TRAP), l'acide 2-[bis[bis(carboxyméthyl)amino]éthyl]amino]acétique (DTPA), et leurs dérivés.

8. Procédé selon l'une quelconque des revendications 1 à 7, dans lequel ledit agent neutralisant est un sel de sodium ou de potassium d'acide formique, ou un sel de sodium ou de potassium d'acide ascorbique, dans lequel, de préférence, lorsque l'agent neutralisant est un sel alcalin ou alcalino-terreux d'acide ascorbique, le sel de gallium et la curcumine ne sont pas présents dans la solution.

9. Kit de préparation d'un complexe de ⁶⁸Ga comprenant les réactifs nécessaires à la réalisation de la réaction de complexation selon l'une quelconque des revendications 1 à 8, dans des récipients unidoses séparés, et des instructions pour l'opérateur qui réalisera la réaction de complexation.

10. Kit selon la revendication 9, comprenant :
- une fiole contenant une molécule fonctionnalisée par un chélateur, un agent neutralisant, de l'acide ascorbique et/ou un sel de celui-ci, un sel de Ga3+ et/ou de la curcumine ou des dérivés de celle-ci ;
- une fiole ou une seringue contenant une solution stabilisante ;
- éventuellement, une fiole ou une seringue contenant une solution d'acide inorganique.

11. Kit selon la revendication 9, comprenant :
- une fiole contenant une molécule fonctionnalisée par un chélateur ;
- une fiole ou une seringue contenant un agent neutralisant, de l'acide ascorbique et/ou un sel de celui-ci, un sel de Ga3+ et/ou de la curcumine ou des dérivés de celle-ci ;
- une fiole ou une seringue contenant une solution stabilisante ; - éventuellement, une fiole ou une seringue contenant une solution d'acide inorganique.

12. Kit selon la revendication 9, comprenant :
- une fiole contenant une molécule fonctionnalisée par un chélateur, un sel alcalin ou alcalino-terreux d'acide ascorbique et éventuellement un sel de Ga³⁺ et/ou de curcumine ou des dérivés de celle-ci ;
- une fiole ou une seringue contenant une solution stabilisante ;
- éventuellement, une fiole ou une seringue contenant une solution d'acide inorganique.

13. Kit selon la revendication 9, comprenant :
- une fiole contenant une molécule fonctionnalisée par un chélateur et un sel alcalin ou alcalino-terreux d'acide ascorbique ;
- éventuellement une fiole contenant un sel de Ga³⁺ et/ou de la curcumine ou des dérivés de celle-ci ;
- une fiole ou une seringue contenant une solution stabilisante ;
- éventuellement, une fiole ou une seringue contenant une solution d'acide inorganique.

14. Utilisation du kit selon les revendications 9 à 13 dans un module de synthèse automatique.
